# EUROPÄISCHE PATENTSCHRIFT

(11) **EP 1 977 723 B1**
(45) Veröffentlichungstag und Bekanntmachung des Hinweises auf die Patenterteilung: **17.02.2010**
(21) Anmeldenummer: 07006908.3
(22) Anmeldetag: 03.04.2007
(51) Int. Cl.: A61F 5/37

(54) **Orthopädische Weste**
Orthopaedic jacket
Gilet orthopédique

(43) Veröffentlichungstag der Anmeldung: 08.10.2008
(73) Patentinhaber: Forni Medical GmbH, 9214 Kradolf (CH)
(72) Erfinder: Pla, Miguel, 8603 Schwerzenbach (CH)
(74) Vertreter: Schaad, Balass, Menzl & Partner AG

(56) Entgegenhaltungen:
- EP-A- 0 898 949
- EP-A1- 0 694 296
- WO-A-82/03767
- US-B1- 6 453 904

## Beschreibung

Die vorliegende Erfindung betrifft eine orthopädische Weste zur Positionierung und Ruhigstellung einer verletzten oberen Extremität, insbesondere der Schulter, gemäss dem Oberbegriff des Anspruchs 1.

Es sind bereits zahlreiche solcher orthopädischen Westen vorgeschlagen worden. Beispielsweise wird in WO-A-82/03767 eine orthopädische Weste beschrieben bestehend aus einer Hauptbahn und drei Hilfsbahnen, wobei die Gesamtheit der Bahnen aus einem Material hergestellt ist, dessen mindestens eine Fläche die Rolle eines weiblichen Befestigungsmittels eines selbsthaftenden Systems spielt, während der äussere Rand jeder Bahn männliche Befestigungsmittel des gleichen Systems aufweist.

Nachteilig an solchen Westen ist, dass sie sich der individuellen Körperform des Patienten nicht anpassen können, was, wenn der Patient über keine optimale Figur verfügt, dazu führt, dass die Weste nicht optimal anliegt. Dies hat nachteilige Auswirkungen bei der Stabilisierung der oberen Extremitäten.

Aufgabe der vorliegenden Erfindung ist es daher, eine orthopädische Weste bereitzustellen, die bei jeder Figur optimal anliegt sowie einfach in der Handhabung ist.

Die Aufgabe wird gelöst durch eine orthopädische Weste gemäss Anspruch 1. Weitere bevorzugte Ausführungsformen sind Gegenstand der abhängigen Ansprüche 2 bis 10.

Die erfindungsgemässe orthopädische Weste weist ein Bauchteil auf, das über ein Schulterteil mit dem Rückenteil verbunden ist. Das Rückenteil weist dabei einen Bauchverschluss auf, welcher mit einem am Bauchteil fixierbaren Verschlusselement versehen ist. Das Bauchteil weist ein Unterarmhalteelement mit einem am Bauchteil fixierbaren Verschlusselement auf. Am Schulterteil ist eine an dem Unterarmhalteelement fixierbare Ellbogenstütze angebracht. Das Rückenteil weist ausserdem eine an dem Unterarmhalteelement fixierbare Oberarmstütze auf. Das Verschlusselement des Bauchverschlusses ist horizontal, d.h. waagrecht zur Oberarmrichtung zweigeteilt und das Verschlusselement des Unterarmhalteelements ist senkrecht, d.h. parallel zur Oberarmrichtung zweigeteilt. Die Zweiteilung dieser beiden Verschlusselemente ermöglicht eine optimale Anpassung an die individuelle Körperform des Patienten. Eine erfindungsgemässe orthopädische Weste kann daher sowohl von sehr schlanken als auch von korpulenten Patienten getragen werden, ohne dass die Stabilisierung der Schulter negativ beeinträchtigt wird.

In einer bevorzugten Ausführungsform der vorliegenden Erfindung weist das Verschlusselement des Bauchverschlusses eine Ausnehmung unter Ausbildung einer ersten und einer zweiten Zunge aus. Das Verschlusselement des Unterarmhalteelementes weist eine Ausnehmung unter Ausbildung einer dritten und einer vierten Zunge auf. Die Form der Zungen ist optimal, da so eine ausreichende Breite vorhanden ist, um einerseits eine genügend grosse Fläche zum Fixieren des Verschlusselementes zu haben, andererseits ist die Zunge breit genug um eine einfache Handhabung sicherzustellen.

Sämtliche Verschlusselemente der erfindungsgemässen orthopädischen Weste werden auf dem Bauchteil oder auf dem Unterarmhalteelement befestigt. Der Stoff dieser beiden Teile ist dergestalt, dass es ein "weibliches" Mittel aufweist, das im allgemeinen von einem veloursartigen Stoff gebildet ist. Unter einem veloursartigen Stoff wird Gewebe mit aufgeschnittenen Schlingen oder ein Gewirk verstanden, dessen Oberfläche durch Aufrauung eine samtähnliche Qualität annimmt. Die Verschlusselemente weisen "männliche" Mittel auf, die im allgemeinen von einer Kunststoffoberfläche gebildet sind, die eine Vielzahl von kleinen Häkchen aufweisen. Diese männlichen Mittel bilden in Kontakt mit dem veloursartigen Stoff ein Flächenreissverschlusssystem der Art "VELCRO" aus.

Das Bauchteil mit dem seitlichen Lappen, das Unterarmhalteelement, die Oberarmstütze sowie sämtliche Verschlusselemente weisen vorzugsweise 40%-60% Baumwolle und 40%-60% Polyester auf. In einer besonders bevorzugten Ausführungsform weisen sie 50% Baumwolle und 50% Polyester auf.

In einer weiteren bevorzugten Ausführungsform weisen sowohl die Zungen des Bauchverschlusses als auch die Zungen des Verschlusselementes des Unterarmhalteelementes endständig Griffelemente auf. Diese sind vorzugsweise dergestalt, dass die Zunge im Endbereich männliche Mittel aufweisen, die eine Vielzahl von kleinen Häkchen aufweisen. Zur besseren Handhabung können auch die Ellbogenstütze und die Oberarmstütze solche Griffelemente aufweisen.

In einer weiteren bevorzugten Ausführungsform enthält das Rückenteil der erfindungsgemässen orthopädischen Weste einen Stoff aus 90%, besonders bevorzugt 100% reiner Baumwolle. Dies hat den Vorteil, dass diejenige Stelle, an welcher der Patient am meisten Schweiss absondert, mit einer Naturfaser bedeckt ist, die die Feuchtigkeit aufnimmt. Dies erhöht den Tragekomfort der erfindungsgemässen Weste erheblich.

In einer weiteren bevorzugten Ausführungsform enthält die Ellbogenstütze, die den Unterarm zusätzlich stabilisiert, 90%, vorzugsweise 100% Trikot. Dies kann beispielsweise ein Stoff, der Baumwolle und Elastomer enthält, sein. Dadurch ist die Ellbogenstütze elastisch, was eine stärkere Fixierung ermöglicht.

In einer weiteren Ausführungsform enthält das Schulterteil einen Stoff aus 90%, vorzugsweise 100% Baumwolle. Der Tragekomfort wird so bei der verletzten Extremität erhöht.

In einer weiteren Ausführungsform der vorliegenden Erfindung weisen sämtliche männlichen Mittel der Verschlusselemente des Bauchverschlusses, der Unterarmstütze, der Ellbogenstütze sowie der Oberarmstütze ein Gegenstück auf, auf dem sie bei Nichtgebrauch fixierbar sind. Dies kann beispielsweise ein veloursartiges Gegenstück oder aber ein weibliches Mittel, d.h. ein klassischer Klettverschluss sein. Die Gegenstücke können die unmittelbare Verlängerung der männlichen Mittel sein, sodass diese bei Nichtgebrauch nur umgeklappt werden müssen. Dies ermöglicht, dass die erfindungsgemässe orthopädische Weste problemlos gewaschen werden kann.

Weitere Merkmale, Einzelheiten und Vorzüge der Erfindung ergeben sich aus den nachfolgend beschriebenen Zeichnungen.

Es zeigt:
- Fig. 1: eine erste Ausführungsform der orthopädischen Weste der vorliegenden Erfindung in flach ausgebreitetem Zustand
- Fig. 2: schematisch das Zusammenwirken der verschiedenen Elemente der orthopädischen Weste
- Fig. 3: eine Detailansicht des Bauchverschlusses der orthopädischen Weste
- Fig. 4: eine Detailansicht der Ellbogenstütze der orthopädischen Weste
- Fig. 5: eine Detailansicht des Unterarmhalteelementes der orthopädischen Weste

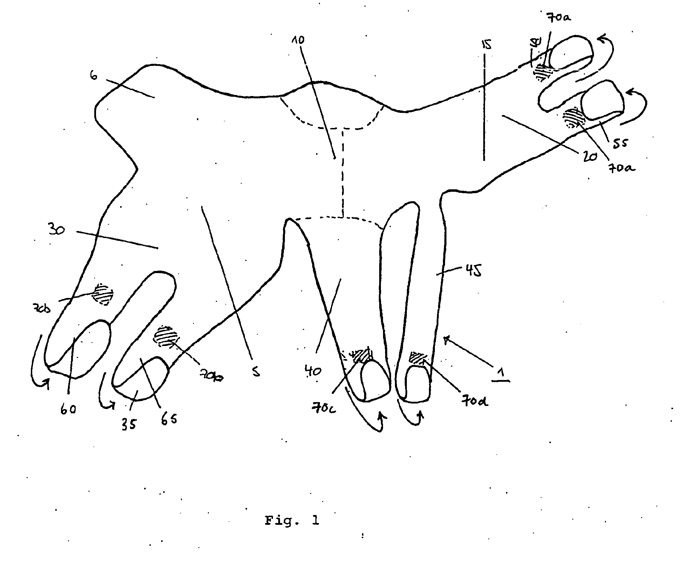

Figur 1 zeigt eine orthopädische Weste 1 in flach ausgebreitetem Zustand. Die orthopädische Weste weist ein Bauchteil 5 auf, das über ein Schulterteil 10 mit einem Rückenteil 15 verbunden ist. Das Bauchteil 5 erstreckt sich flächig von der Schulter über die Brust bis zum Bauch und weist einen seitlichen Lappen 6 auf, der sich seitlich bis über die Rippen erstreckt. Das Rückenteil 15 weist einen Bauchverschluss 20 auf, welcher mit einem am Bauchteil fixierbaren Verschlusselement 25 versehen ist. Dieser Bauchverschluss 20 erstreckt sich im am Patienten angezogenen Zustand über den seitlichen Lappen 6 und wird je nach Umfang des Patienten mehr oder weniger seitlich am Bauchteil 5 respektive am seitlichen Lappen 6, der erfindungsgemäss als zugehörig zum Bauchteil 5 betrachtet wird, fixiert. Das Bauchteil 5 weist ausserdem ein Unterarmhalteelement 30 mit einem am Bauchteil fixierbaren Verschlusselement 35 auf. Ausserdem weist die erfindungsgemässe Weste 1 eine mit dem Schulterteil 10 verbundene Ellbogenstütze 40 auf, die an dem Unterarmhalteelement 30 fixierbar ist. Die Ellbogenstütze 40 unterstützt das Unterarmhalteelement 30 zusätzlich und erhöht damit die Stabilisierung des Schulterbereichs. Schliesslich ist eine Oberarmstütze 45 mit dem Rückenteil verbunden, die ebenfalls am Unterarmhalteelement 30 fixierbar ist. Das Verschlusselement 25 des Bauchverschlusses 20 ist horizontal, d.h. waagrecht zur Oberarmrichtung, zweigeteilt, wobei vorzugsweise eine erste Zunge 50 und eine zweite Zunge 55 ausgebildet werden, sodass eine Anpassung an die Figur des Patienten problemlos möglich ist, ohne dass es zu Falten in der Weste kommt, die die Stabilisierung der Schulter beeinträchtigen können. Das Verschlusselement 35 des Unterarmhalteelements 30 ist senkrecht, d.h. parallel zur Oberarmrichtung zweigeteilt, wobei vorzugsweise eine dritte Zunge 60 und eine vierte Zunge 65 ausgebildet werden. Auch die dritte Zunge 60 und die vierte Zunge 65 ermöglichen eine optimale Anpassung an die Figur des Patienten, sei es bezüglich des Bauchumfangs oder aber der Rumpflänge. Durch die beiden Zungen ist eine Idealstellung des Unterarms, d.h. im 90° Winkel zum Oberarm, problemlos möglich. Sämtliche Verschlusselemente, d.h. das Verschlusselement des Bauchverschlusses, das Verschlusselement des Unterarmhalteelementes, das Verschlusselement der Ellbogenstütze sowie das Verschlusselement der Oberarmstütze weisen sogenannte "männliche" Mittel 70a, 70b, 70c und 70d auf. Darunter wird eine Vielzahl von kleinen Häkchen verstanden, die im allgemeinen von einer Kunststoffoberfläche gebildet sind. Es handelt sich dabei um den Hakenteil von Klett- oder Velcroverschlüssen. Diese Verschlusselemente werden alle entweder auf dem Bauchteil 5, respektive dessen seitlichen Lappen 6 oder auf dem Unterarmhalteelement 30 fixiert, der aus einem veloursartigen Stoff, vorzugsweise aus 40%-60% Baumwolle und 40%-60% Polyester, besonders bevorzugt 50% Baumwolle und 50% Polyester gebildet ist.

Auf der jeweiligen Unterseite respektive Oberseite des Stoffes können zusätzlich zu den männlichen Mitteln 70a, 70b, 70c und 70 d weitere männliche Mittel angeordnet sein, die die Ausbildung eines Griffelementes 51, 56, 61, 68, 75a und 75b ermöglichen. Dabei werden die Verschlusselemente, die alle vorzugsweise aus einem veloursartigen Stoff sind, endständig umgeklappt, wobei die männlichen Mittel und der veloursartige Stoff eine feste Bindung ausbilden. Durch die Griffelemente ist die orthopädische Weste einfacher in der Handhabung.

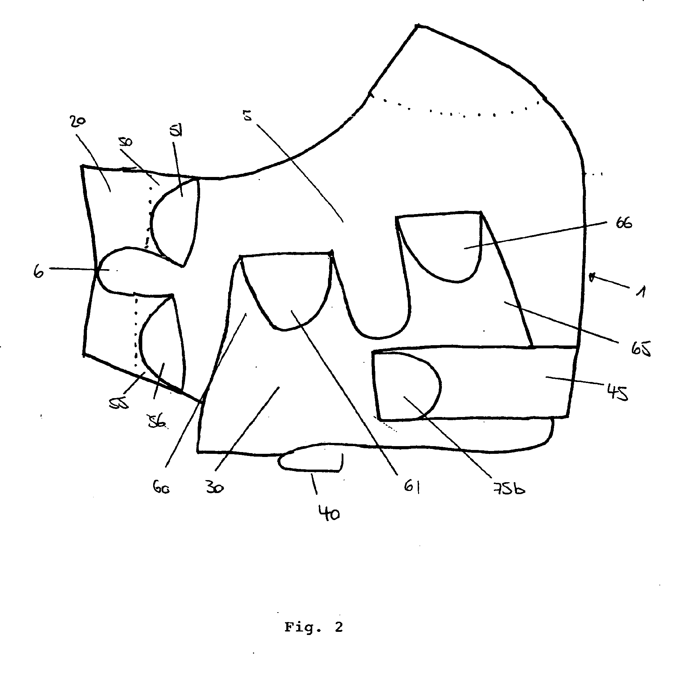

Figur 2 zeigt schematisch das Zusammenwirken der verschiedenen Elemente der orthopädischen Weste 1. Auf dem Bauchteil 5 mit dem seitlichen Lappen 6 ist der Bauchverschluss 20 fixiert, dessen Verschlusselement 25 eine Ausnehmung unter Ausbildung einer ersten Zunge 50 und einer zweiten Zunge 55 aufweist. Die erste Zunge 50 und die zweite Zunge 55 sind mittels männlicher Mittel 70a auf dem veloursartigen Stoff des Bauchteils 5 unter Ausbildung eines Flächenreissverschlusssystems der Art "VELCRO" fixiert. Auf der dem Bauchteil 5 abgewandten Seite weisen die erste Zunge 50 und die zweite Zunge 55 weitere männliche Mittel auf, sodass durch Umklappen der ersten Zunge 50, respektive der zweiten Zunge 55 die Griffelemente 51 und 56 ausgebildet werden.

Das Unterarmhalteelement 30 ist am Bauchteil 5 mittels des Verschlusselementes 35 befestigt. Das Verschlusselement 35 liegt in Form einer dritten Zunge 60 und einer vierten Zunge 65 vor. Auch die dritte Zunge 60 und die vierte Zunge 65 sind mittels männlicher Mittel 70b auf dem veloursartigen Stoff des Bauchteils 5 unter Ausbildung eines Flächenreissverschlusssystems der Art "VELCRO" fixiert. Auf der dem Bauchteil 5 abgewandten Seite weisen die dritte Zunge 60 und die vierte Zunge 65 weitere männliche Mittel auf, sodass durch Umklappen der dritten Zunge 60 und der vierten Zunge 65 die Griffelemente 61 und 66 ausgebildet werden. Schematisch wird die Ellbogenstütze 40 angedeutet. Die Oberarmstütze 45 wird ebenfalls mittels männlicher Mittel auf dem Unterarmhalteelement 30 fixiert und ist ebenfalls mit einem Griffelement 75b versehen.

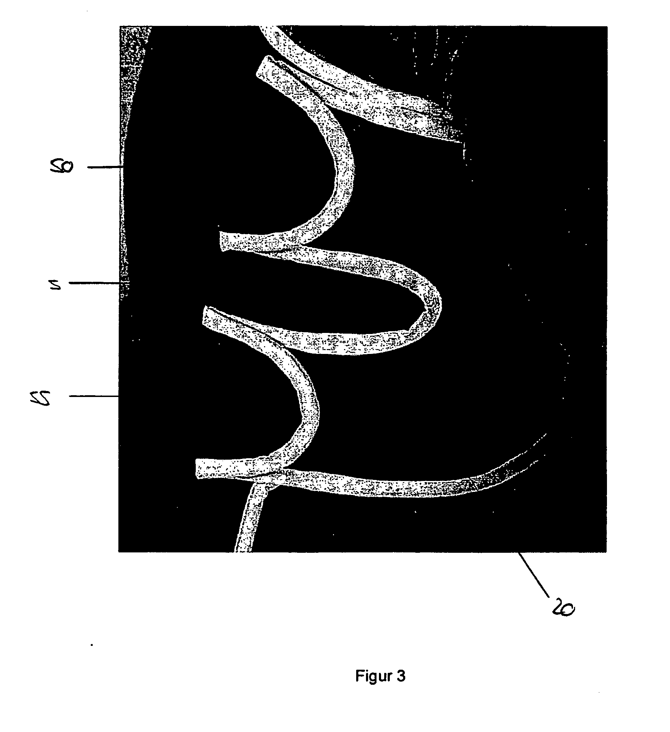

Figur 3 zeigt eine detaillierte Ansicht des Bauchverschlusses 20 im getragenen Zustand der Weste, bei dem die erste Zunge 50 und die zweite Zunge 55 am Bauchteil fixiert sind. Je nach Statur des Patienten werden die Zungen parallel oder schräg am Bauchteil fixiert. Ebenso hängt es von der Statur des Patienten ab, ob die Zungen eher seitlich oder in der Mitte des Bauchteils angeordnet werden.

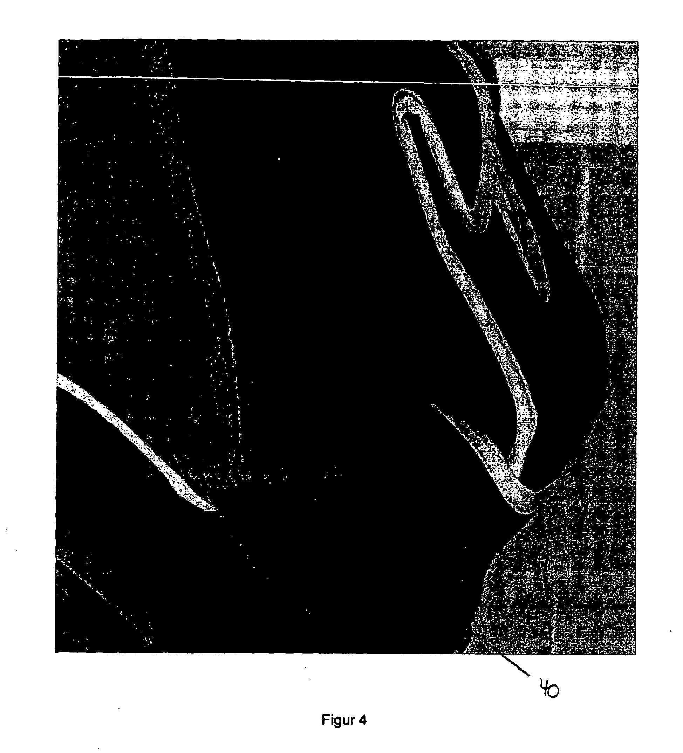

Figur 4 zeigt eine detaillierte Ansicht der Ellbogenstütze 40. Die Ellbogenstütze 40 enthält vorzugsweise 100% Trikot. Dadurch wird sie elastisch, was eine stärkere Fixierung der Schulter ermöglicht.

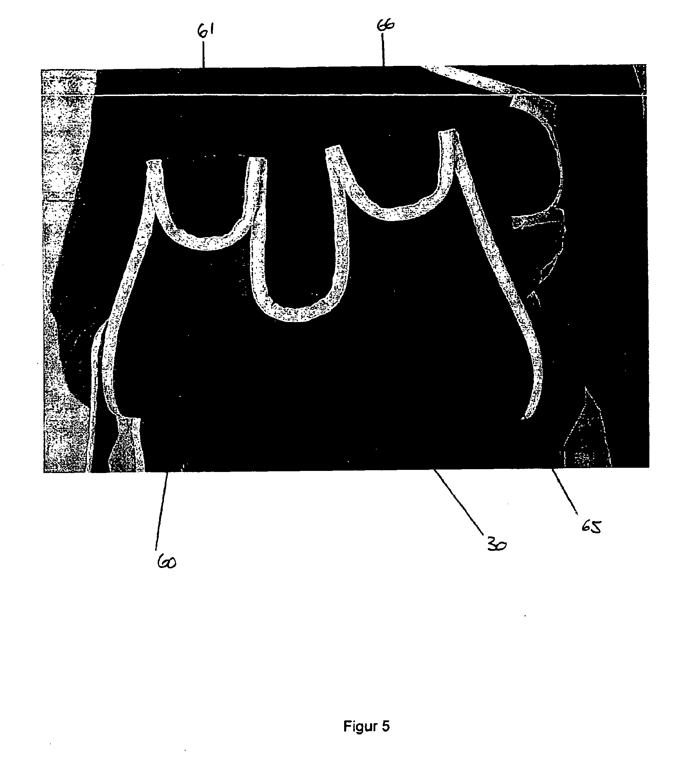

Figur 5 zeigt eine detaillierte Ansicht des Unterarmhalteelementes 30 vor der Befestigung der Ellbogenstütze und der Oberarmstütze. Dabei sind die dritte Zunge 60 und die vierte Zunge 65 mittels männlicher Mittel am Bauchteil 5 unter Ausbildung eines Flächenreissverschlusssystems der Art "VELCRO" fixiert. Ebenfalls sichtbar sind die Griffelemente 61 und 66.

## Patentansprüche

1. Orthopädische Weste (1) mit
einem Bauchteil (5), das über ein Schulterteil (10) mit einem Rückenteil (15) verbunden ist, wobei das Rückenteil (15) einen Bauchverschluss (20) aufweist, welcher mit einem am Bauchteil (5) fixierbaren Verschlusselement (25) versehen ist, und das Bauchteil (5) ein Unterarmhalteelement (30) mit einem am Bauchteil (5) fixierbaren Verschlusselement (35) aufweist,
einer mit dem Schulterteil (10) verbundenen an dem Unterarmhalteelement (30) fixierbare Ellbogenstütze (40), und
einer mit dem Rückenteil (15) verbundenen an dem Unterarmhalteelement (30) fixierbaren Oberarmstütze (45), **dadurch gekennzeichnet, dass**
das Verschlusselement (25) des Bauchverschlusses (20) im wesentlichen waagrecht zur Oberarmrichtung zweigeteilt ist, und
das Verschlusselement (35) des Unterarmhalteelements (30) im wesentlichen parallel zur Oberarmrichtung zweigeteilt ist.

2. Orthopädische Weste (1) gemäss Anspruch 1, **dadurch gekennzeichnet, dass** das Verschlusselement (25) des Bauchverschlusses (20) eine Ausnehmung unter Ausbildung einer ersten (50) und einer zweiten Zunge (55) und das Verschlusselement (35) des Unterarmhalteelements (30) eine Ausnehmung unter Ausbildung einer dritten (60)und einer vierten Zunge (65) aufweist.

3. Orthopädische Weste (1) gemäss Anspruch 2, **dadurch gekennzeichnet, dass** die Zungen (50, 55, 60, 65) endständig Griffelemente (51, 56, 61, 66) aufweisen.

4. Orthopädische Weste gemäss einem der vorangehenden Ansprüche, **dadurch gekennzeichnet, dass** die Ellbogenstütze (40) und die Oberarmstütze (45) je ein Verschlusselement mit einem Griffelement aufweist.

5. Orthopädische Weste (1) gemäss einem der vorangehenden Ansprüche, **dadurch gekennzeichnet, dass** das Rückenteil aus einem Stoff enthaltend 90%, vorzugsweise 100% Baumwolle gebildet ist.

6. Orthopädische Weste (1) gemäss einem der vorangehenden Ansprüche, **dadurch gekennzeichnet, dass** die Ellbogenstütze aus einem Stoff enthaltend 90%, vorzugsweise 100% Trikot gebildet ist.

7. Orthopädische Weste (1) gemäss einem der vorangehenden Ansprüche, **dadurch gekennzeichnet, dass** das Schulterteil aus einem Stoff enthaltend 90%, vorzugsweise 100% Baumwolle gebildet ist.

8. Orthopädische Weste (1) gemäss einem der vorangehenden Ansprüche, **dadurch gekennzeichnet, dass** der Bauchteil, das Unterarmhalteelement, die Oberarmstütze sowie die Verschlusselemente aus einem Stoff enthaltend 40-60% Baumwolle und 40-60% Polyester, vorzugsweise 50% Baumwolle und 50% Polyester gebildet sind.

9. Orthopädische Weste (1) gemäss einem der vorangehenden Ansprüche, **dadurch gekennzeichnet, dass** die Verschlusselemente und die Griffelemente männliche Mittel enthalten, und die Oberflächen, die dazu bestimmt sind, mit den Verschlusselementen oder den Griffelementen in Kontakt zu sein, dergestalt sind, dass sie mit den männlichen Mitteln ein selbsthaftendes System ausbilden.

10. Orthopädische Weste (1) gemäss Anspruch 9, **dadurch gekennzeichnet, dass** die männlichen Mittel der Verschlusselemente ein Gegenstück haben, auf dem sie bei Nichtgebrauch fixierbar sind.

## Claims

1. Orthopaedic jacket (1), with
a midriff part (5) connected to a back part (15) via a shoulder part (10), the back part (15) having a midriff closure piece (20) provided with a closure element (25) that can be fixed to the midriff part (5), and the midriff part (5) having a forearm retention element (30) with a closure element (35) that can be fixed to the midriff part (5),
an elbow support (40) that is connected to the shoulder part (10) and can be fixed to the forearm retention element (30), and
an upper arm support (45) that is connected to the back part (15) and can be fixed to the forearm retention element (30), **characterized in that**
the closure element (25) of the midriff closure piece (20) is divided in two substantially horizontally with respect to the direction of the upper arm, and
the closure element (35) of the forearm retention element (30) is divided in two substantially parallel to the direction of the upper arm.

2. Orthopaedic jacket (1) according to Claim 1, **characterized in that** the closure element (25) of the midriff closure piece (20) has a recess forming a first tongue (50) and a second tongue (55), and the closure element (35) of the forearm retention element (30) has a recess forming a third tongue (60) and a fourth tongue (65).

3. Orthopaedic jacket (1) according to Claim 2, **characterized in that** the tongues (50, 55, 60, 65) have grip elements (51, 56, 61, 66) at their ends.

4. Orthopaedic jacket according to one of the preceding claims, **characterized in that** the elbow support (40) and the upper arm support (45) each have a closure element with a grip element.

5. Orthopaedic jacket (1) according to one of the preceding claims, **characterized in that** the back part is made of a material containing 90% cotton, preferably 100% cotton.

6. Orthopaedic jacket (1) according to one of the preceding claims, **characterized in that** the elbow support is made of a material containing 90% tricot, preferably 100% tricot.

7. Orthopaedic jacket (1) according to one of the preceding claims, **characterized in that** the shoulder part is made of a material containing 90% cotton, preferably 100% cotton.

8. Orthopaedic jacket (1) according to one of the preceding claims, **characterized in that** the midriff part, the forearm retention element, the upper arm support and the closure elements are made of a material containing 40-60% cotton and 40-60% polyester, preferably 50% cotton and 50% polyester.

9. Orthopaedic jacket (1) according to one of the preceding claims, **characterized in that** the closure elements and the grip elements comprise male means, and the surfaces that are intended to be in contact with the closure elements or the grip elements are designed in such a way that they form a self-adhesive system with the male means.

10. Orthopaedic jacket (1) according to Claim 9, **characterized in that** the male means of the closure elements have a mating piece to which they can be fixed when not in use.

## Revendications

1. Gilet orthopédique (1) présentant :
une partie ventrale (5) qui est reliée à une partie dorsale (15) par une partie d'épaule (10), la partie dorsale (15) présentant une fermeture ventrale (20) dotée d'un élément de fermeture (25) qui peut être fixé sur la partie ventrale (5), la partie ventrale (5) présentant un élément (30) de maintien d'aisselle doté d'un élément de fermeture (35) qui peut être fixé sur la partie ventrale (5),
un soutien de coude (40) relié à la partie d'épaule (10) et apte à être fixé à l'élément (30) de maintien d'aisselle et
un soutien de bras (45) relié à la partie de dos (15) et apte à être fixé à l'élément (30) de maintien d'aisselle,
**caractérisé en ce que**
l'élément de fermeture (25) de la fermeture ventrale (20) est divisé en deux essentiellement à l'horizontale par rapport à la direction du bras et
l'élément de fermeture (35) de l'élément (30) de maintien d'aisselle est divisé en deux essentiellement en parallèle à la direction du bras.

2. Gilet orthopédique (1) selon la revendication 1, **caractérisé en ce que** l'élément de fermeture (25) de la fermeture ventrale (20) présente une découpe qui forme une première patte (50) et une deuxième patte (55) et **en ce que** l'élément de fermeture (35) de l'élément (30) de soutien d'aisselle présente une découpe qui forme une troisième patte (60) et une quatrième patte (65).

3. Gilet orthopédique (1) selon la revendication 2, **caractérisé en ce que** les pattes (50, 55, 60, 65) présentent à leur extrémité des éléments de saisie (51, 56, 61, 66).

4. Gilet orthopédique selon l'une des revendications précédentes, **caractérisé en ce que** les soutiens de coude (40) et les soutiens de bras (45) présentent chacun un élément de fermeture doté d'un élément de saisie.

5. Gilet orthopédique (1) selon l'une des revendications précédentes, **caractérisé en ce que** la partie dorsale est formée d'une toile qui contient 90 % et de préférence 100 % de coton.

6. Gilet orthopédique (1) selon l'une des revendications précédentes, **caractérisé en ce que** les soutiens de coude sont formés d'une toile qui contient 90 % et de préférence 100 % de tricot.

7. Gilet orthopédique (1) selon l'une des revendications précédentes, **caractérisé en ce que** la partie d'épaule est formée d'une toile qui contient 90 % et de préférence 100 % de coton.

8. Gilet orthopédique (1) selon l'une des revendications précédentes, **caractérisé en ce que** la partie ventrale, la partie de maintien d'aisselle, les soutiens de bras et les éléments de fermeture sont formés d'une toile qui contient de 40 à 60 % de coton et de 40 à 60 % de polyester et de préférence de 50 % de coton et de 50 % de polyester.

9. Gilet orthopédique (1) selon l'une des revendications précédentes, **caractérisé en ce que** les éléments de fermeture et les éléments de saisie contiennent des moyens mâles et **en ce que** les surfaces destinées à entrer en contact avec les éléments de fermeture ou les éléments de saisie sont configurées de manière à former avec les moyens mâles un système auto-adhérent.

10. Gilet orthopédique (1) selon la revendication 9, **caractérisé en ce que** les moyens mâles des éléments de fermeture ont une pièce complémentaire sur laquelle ils peuvent être fixés en cas de non-utilisation.
